# EUROPEAN PATENT APPLICATION

(11) **EP 2 019 098 A1**
(43) Date of publication of application: **28.01.2009**
(21) Application number: 07743479.3
(22) Date of filing: 16.05.2007
(51) Int. Cl.: C07D 311/16, A23L 1/30, A23L 2/00, A61K 31/352, A61K 36/00, A61P 3/04, A61P 3/06, A61P 3/10, A61P 9/10, A61P 9/12, A61P 13/12, A61P 25/00, A61P 29/00, A61P 31/04, A61P 35/04, A61P 37/06, A61P 37/08, A61P 43/00

(54) **SUPPRESSOR OF EXPRESSION OF MCP-1, AND AMELIORATING AGENT FOR INFLAMMATORY DISEASE, PHARMACEUTICAL, SUPPLEMENT, FOOD, BEVERAGE OR FOOD ADDITIVE USING THE SUPPRESSOR**

(30) Priority: 17.05.2006 JP 2006137223
(71) Applicant: Arkray, Inc., Kyoto-shi, Kyoto 601-8045 (JP)
(72) Inventor: SASAKI, Takao, Minami-ku, Kyoto-shi, Kyoto 601-8045 (JP)
(74) Representative: Behnisch, Werner
(86) International application number: PCT/JP2007/060044
(87) International publication number: WO 2007/132893

(57) **Abstract**

A suppressor of the expression of MCP-1 is provided that is excellent in safety and is widely applicable to, for example, foods. The suppressor of the expression of MCP-1 according to the present invention is **characterized by** containing auraptene. Auraptene is contained in citrus fruits such as Hassaku, Amanatsu, Natsumikan, and grapefruit. Since these citrus fruits have been eaten for a long time, the present invention has no problem in safety. Furthermore, even when added to, for example, foods, auraptene does not impair the flavor thereof because it is substantially tasteless and odorless. Since auraptene has a low calorific value, for example, an obese person or a diabetic patient can ingest it over a long period of time. Auraptene suppresses the expression of MCP-1 at a genetic level (see FIG. 1) and a protein level.

## Description

### Technical Field

The present invention relates to suppressors of the expression of MCP-1 as well as ameliorating agents for inflammatory diseases, pharmaceuticals, supplements, foods, beverages, and food additives using the suppressors.

### Background Art

MCP-1 (monocyte chemoattractant protein-1) is a type of chemokine. This is secreted mainly from, for example, monocytes, macrophages, fibroblasts, and vascular endothelial cells and has functions such as monocyte or T-cell migration activity, induction of cytokine production, and adhesion molecule expression. The MCP-1 receptor is CCR2. Recent research showed that adipose tissue is an organ that secretes MCP-1 and an increased amount of MCP-1 is secreted in the adipose tissue of an obese person. In addition, MCP-1 is known to be associated with, for example, insulin resistance as well as inflammatory diseases such as infections, autoimmune diseases, rheumatoid arthritis, multiple sclerosis, organ transplant rejection, atherosclerosis, and cancer metastasis (Nonpatent Document1). Therefore the development of pharmaceuticals that target MCP-1 is being promoted (Patent Document1). However, even when having significant effects, chemically-synthesized pharmaceuticals can be accompanied by, for instance, adverse effects and therefore have problems in terms of safety. Furthermore, since, for example, insulin resistance and inflammatory diseases including obesity are closely related to lifestyle, exercise and improvements in dietary habits are required. Accordingly, it is conceivable that suppression of the expression of MCP-1 in daily living including, for example, diet, leads to prevention or treatment of, for example, inflammatory diseases. However, as described above, since the chemically-synthesized pharmaceuticals have problems of adverse effects, long-term ingestion thereof is difficult. Furthermore, the chemically-synthesized pharmaceuticals often have unique flavors. Therefore, when they are ingested together with foods, for instance, they impair the original flavors of the foods, which also is a problem. Accordingly, the chemically-synthesized pharmaceuticals may be useful in, for example, the treatment under the direction of a medical doctor but have a problem in safety. Moreover, they are not applicable to, for example, foods, beverages, and supplements, and therefore have a problem in that they cannot be applied widely.
Nonpatent Document 1: "Himan Kenkyu (Study of Obesity)" Vol. 11 No.3 2005 27(267)-32(272)
Patent Document 1: JP 2005-187451 A

### Disclosure of Invention

The present invention, therefore, is intended to provide a suppressor of the expression of MCP-1 that is excellent in safety and can be applied widely to, for example, a food.

In order to achieve the aforementioned object, a suppressor of the expression of MCP-1 according to the present invention contains auraptene.

An ameliorating agent for an inflammatory disease of the present invention contains a suppressor of the expression of MCP-1 according to the present invention.

Furthermore, the present invention relates to a pharmaceutical for preventing or treating an inflammatory disease, and the pharmaceutical contains an ameliorating agent for an inflammatory disease of the present invention.

The present invention relates to a supplement, food, beverage, or food additive for preventing or ameliorating an inflammatory disease, and they each contain an ameliorating agent for an inflammatory disease of the present invention.

The present invention relates to a method of suppressing the expression of MCP-1, and the method includes a step of administering auraptene. The present invention also relates to a method of preventing or treating an inflammatory disease, and the method includes a step of administering auraptene.

The present invention relates to use of auraptene for suppressing the expression of MCP-1 or preventing or treating an inflammatory disease.

In order to achieve the aforementioned object, the inventor made a series of studies on substances that suppress the expression of MCP-1, particularly natural products. As a result, auraptene contained in citrus fruits was found to suppress the expression of MCP-1, which resulted in the present invention. Citrus fruits containing auraptene have been used as foods for a long time and therefore have no problem in safety. Furthermore, auraptene has no or very little unique flavor, if any. Accordingly, even when used for foods or beverages, auraptene does not impair the flavors thereof. Moreover, auraptene has a low calorific value, due to which there is no problem even when a person who needs to control calories, such as an obese person or a diabetic patient, ingests it over a long period of time. Thus the suppressor of the expression of MCP-1 according to the present invention is excellent in safety, can be applied widely to, for example, foods, and can be ingested over a long period of time.

### Brief Description of Drawings

FIG. 1 is a graph showing the effect of suppressing the expression of MCP-1 at a genetic level in an example of the present invention.
FIG. 2 is a graph showing the effect of suppressing the expression of MCP-1 at a protein level in the aforementioned example.

### Best Mode for Carrying Out the Invention

In the present invention, the suppression of the expression of MCP-1 described above is at least one of suppression of the expression at a genetic level and that at a protein level. The suppression of the expression at a genetic level includes suppression of transcription to mRNA. Furthermore, the suppression of the expression at a protein level includes, for example, translational suppression as well as suppression of modification when the modification occurs after translation.

In the present invention, it is preferable that the aforementioned auraptene be derived from a citrus fruit. The citrus fruit-derived auraptene may be derived from any part of the citrus fruit, and examples thereof include one derived from fruit juice, one derived from pulp, and one derived from pericarp. The citrus fruit is not particularly limited and examples thereof include Amanatsu (Latin scientific name: Citrus natsudaidai HAYATA), Hassaku (Latin scientific name: Citrus hassaku Hort. ex TANAKA), Natsumikan (Latin scientific name: Citrus natsudaidai HAYATA, variable species), and grapefruit (Latin scientific name: Citrus paradisi).
Furthermore, the auraptene to be used may be, for example, one that has been isolated from a citrus fruit and has been purified or may be in the crude state containing the whole or a part of a citrus fruit. Moreover, the auraptene to be used may be a synthetic product or a commercial product.

The composition and form of the suppressor of the expression of MCP-1 according to the present invention are not limited as long as the suppressor contains auraptene as described above. That is, in the present invention, the suppressor of the expression of MCP-1 may be auraptene alone or further may contain, for example, other components. The other components are not limited and can be determined suitably according to the intended use of the present invention. Examples thereof include various components described below. For example, administration or ingestion of the suppressor of the expression of MCP-1 according to the present invention allows the expression of MCP-1 to be suppressed at, for example, a genetic level or a protein level. The suppressor of the expression according to the present invention is not limited, but preferably it is administered to or ingested by, for example, humans and mammals other than humans.

The suppressor of the expression of MCP-1 according to the present invention is applicable to, for example, ameliorating agents for inflammatory diseases, pharmaceuticals, supplements, foods, beverages, and food additives. In this case, for example, the compositions and forms thereof also are not limited as in the case of the suppressor of the expression of MCP-1 according to the present invention.

The ameliorating agent for an inflammatory disease of the present invention contains the suppressor of the expression of MCP-1 according to the present invention described above. Examples of the inflammatory disease include metabolic syndrome, allergy, renal disease, infection, autoimmune disease, rheumatoid arthritis, multiple sclerosis, organ transplant rejection, and cancer metastasis. Examples of the metabolic syndrome include insulin resistance, hyperinsulinemia, type 2 diabetes mellitus, hyperlipidemia, arteriosclerosis, hypertension, obesity, and visceral fat accumulation. Administration of the ameliorating agent for an inflammatory disease of the present invention makes it possible to ameliorate such inflammatory diseases. The subjects to which it is administered are not limited and can be, for example, humans and mammals other than humans.

In the present invention, the amount of auraptene to be administered or ingested is not limited. Furthermore, auraptene may be administered or ingested, for example, once a day or a few times a day.

The pharmaceutical of the present invention is a pharmaceutical for preventing or treating an inflammatory disease and contains the ameliorating agent for an inflammatory disease of the present invention described above. Administration of the pharmaceutical according to the present invention makes it possible to prevent or treat, for example, metabolic syndromes such as insulin resistance, hyperinsulinemia, type 2 diabetes mellitus, hyperlipidemia, arteriosclerosis, hypertension, obesity, and visceral fat accumulation, as well as diseases such as allergy, renal disease, infection, autoimmune disease, rheumatoid arthritis, multiple sclerosis, organ transplant rejection, and cancer metastasis. The subjects to which the pharmaceutical of the present invention is administered are not limited and can be, for example, humans and mammals other than humans.

The pharmaceutical of the present invention may contain, for example, other components for suppressing the expression of MCP-1 and pharmaceutically allowable additives in addition to the ameliorating agent of the present invention described above. Specific examples of the dosage form of the pharmaceutical according to the present invention include a tablet, subtle granules (including powders), a capsule, and a liquid (including a syrup). According to these respective dosage forms, for example, additives or base materials that are suitable thereto, respectively, are used suitably, and they can be produced according to a conventional method described in, for example, Pharmacopeia of Japan. Furthermore, the administration route is not particularly limited and, for example, can be oral administration or parenteral administration. Examples of the parenteral administration include intraoral administration, tracheobronchial administration, rectal administration, subcutaneous administration, intramuscular administration, and intravenous administration.

The supplement of the present invention is a supplement for preventing or ameliorating an inflammatory disease and contains the ameliorating agent for an inflammatory disease of the present invention. Ingestion of the supplement according to the present invention makes it possible to prevent or ameliorate, for example, metabolic syndromes such as insulin resistance, hyperinsulinemia, type 2 diabetes mellitus, hyperlipidemia, arteriosclerosis, hypertension, obesity, and visceral fat accumulation as well as diseases such as allergy, renal disease, infection, autoimmune disease, rheumatoid arthritis, multiple sclerosis, organ transplant rejection, and cancer metastasis. The supplement of the present invention is not limited but preferably is ingested by humans and mammals other than humans as in the aforementioned cases. The supplement of the present invention may contain, for example, various additives and other supplements in addition to the ameliorating agent of the present invention described above. Examples of the other components include other components for suppressing the expression of MCP-1, various vitamins such as vitamin C, amino acid, and oligosaccharide. The form of the supplement of the present invention is not particularly limited and examples thereof include a tablet, subtle granules (including powders), a capsule, and a liquid (including a syrup).

The food of the present invention is a food for preventing or ameliorating an inflammatory disease and contains the ameliorating agent for an inflammatory disease of the present invention described above. Ingestion of the food according to the present invention makes it possible to prevent or ameliorate, for example, metabolic syndromes such as insulin resistance, hyperinsulinemia, type 2 diabetes mellitus, hyperlipidemia, arteriosclerosis, hypertension, obesity, and visceral fat accumulation as well as diseases such as allergy, renal disease, infection, autoimmune disease, rheumatoid arthritis, multiple sclerosis, organ transplant rejection, and cancer metastasis. The food of the present invention is not limited but preferably is ingested by, for example, humans and mammals other than humans. The food of the present invention may contain, for example, various components in addition to the aforementioned ameliorating agent. Examples of the other components include other components for suppressing the expression of MCP-1, various additives, and supplements. In the present invention, the aforementioned food includes, for example, both a common food and a functional food. In the present invention, the form of the food is not limited and examples thereof include cereals, noodles, confectioneries, soups, meats, seafoods, seaweeds, vegetables, fruits, legumes, various processed products, dried foods, frozen foods, smoked products, canned foods, bottled foods, and retort foods.

The beverage of the present invention is a beverage for preventing or ameliorating an inflammatory disease and contains the ameliorating agent for an inflammatory disease of the present invention described above. Ingestion of the beverage according to the present invention makes it possible to prevent or ameliorate, for example, metabolic syndromes such as insulin resistance, hyperinsulinemia, type 2 diabetes mellitus, hyperlipidemia, arteriosclerosis, hypertension, obesity, and visceral fat accumulation as well as diseases such as allergy, renal disease, infection, autoimmune disease, rheumatoid arthritis, multiple sclerosis, organ transplant rejection, and cancer metastasis. The beverage of the present invention is not limited but preferably is ingested by, for example, humans and mammals other than humans. The beverage of the present invention may contain, for example, various components in addition to the aforementioned ameliorating agent. Examples of the other components include other components for suppressing the expression of MCP-1, various additives, and supplements. In the present invention, the beverage includes, for example, both a common beverage and a functional beverage. In the present invention, the form of the beverage is not limited and examples thereof include refreshing beverages such as juices, carbonated beverages, coffees, teas, green teas, and mineral waters.

The food additive of the present invention is a food additive for preventing or ameliorating an inflammatory disease and contains the ameliorating agent for an inflammatory disease of the present invention described above. Ingestion of the food additive according to the present invention makes it possible to prevent or ameliorate, for example, metabolic syndromes such as insulin resistance, hyperinsulinemia, type 2 diabetes mellitus, hyperlipidemia, arteriosclerosis, hypertension, obesity, and visceral fat accumulation as well as diseases such as allergy, renal disease, infection, autoimmune disease, rheumatoid arthritis, multiple sclerosis, organ transplant rejection, and cancer metastasis. The food additive of the present invention is not limited but preferably is ingested by, for example, humans and mammals other than humans. The food additive of the present invention may contain, for example, various components in addition to the aforementioned ameliorating agent. Examples of the other components include other components for suppressing the expression of MCP-1, various additives, and supplements. The form of the food additive according to the present invention is not limited and examples thereof include liquids, powders, flakes, and granules. The food additive of the present invention also includes food additives for beverages in addition to foods.

The present invention relates to a method of suppressing the expression of MCP-1, and the method includes a step of administering the aforementioned auraptene. Administration of auraptene makes it possible to suppress the expression of MCP-1 in a cell. The subjects to which auraptene is administered are not limited and may be, for example, biological bodies or cells isolated from biological bodies. The biological bodies are not limited and can be, for example, humans or mammals including humans. The form of the auraptene to be administered is not limited and examples thereof include similar forms to, for example, those of the suppressor of the expression of MCP-1, ameliorating agent for an inflammatory disease, pharmaceutical, supplement, food, beverage, and food additive of the present invention described above.

The aforementioned administration method is not limited and can be, for example, oral administration or parenteral administration in the case of a biological body. Examples of the parenteral administration include intraoral administration, tracheobronchial administration, rectal administration, subcutaneous administration, intramuscular administration, and intravenous administration. Furthermore, in the case of a cell isolated from a biological body, examples of the administration method include a method in which auraptene is added to a culture medium and thereby is cultured.

The present invention relates to a method of preventing or treating an inflammatory disease, and the method includes a step of administering the suppressor of the expression of MCP-1 according to the present invention. The form of the suppressor of the expression of MCP-1 according to the present invention is not limited and is the same as described above. Furthermore, the suppressor of the expression of MCP-1 may be administered as an ameliorating agent for an inflammatory disease, pharmaceutical, supplement, food, beverage, or food additive as described above.

The administration method is not limited and examples thereof include oral administration or parenteral administration. Examples of the parenteral administration include intraoral administration, tracheobronchial administration, rectal administration, subcutaneous administration, intramuscular administration, and intravenous administration. The subjects to which the suppressor of the expression of MCP-1 according to the present invention is administered are not limited and can be, for example, humans or mammals including humans.

Preferably, the auraptene of the present invention is produced using citrus fruit as a raw material as described above. An example (JP11(1999)-29565 A) of this production method is described below.

First, pericarp of a citrus fruit is immersed in water at ordinary temperature and then was centrifuged. Thus pericarp oil is obtained. Fruit, pulp, or fruit juice may be used instead of the pericarp, but the use of pericarp is preferable since it has a high auraptene content.

Subsequently, the pericarp oil is allowed to be adsorbed on an adsorbent. Preferably, the adsorbent to be used is, for example, a porous adsorbent that is electrically neutral and has a large specific surface area. Examples of such an adsorbent include resin containing a copolymer of styrene and divinylbenzene. Furthermore, from the viewpoint of purifying auraptene efficiently through an increase in the adsorption amount, it is preferable that the resin to be used as the adsorbent be used in the dry state.

The adsorbent with the pericarp oil adsorbed thereon is washed using an aqueous alcohol solution. This removes the impurities adsorbed on the adsorbent. The alcohol concentration of the aqueous alcohol solution is, for example, lower than 50% (volume ratio), preferably 10% to 45%, and more preferably 35% to 45%. Examples of the alcohol include ethanol and isopropyl alcohol. Particularly, when purified auraptene is used, for example, for supplements, foods, beverages, or food additives, ethanol is preferred, while when it is used for pharmaceuticals, isopropyl alcohol is preferred. Moreover, the amount of the aqueous alcohol solution is not limited but is preferably, for example, about 14 times (volume ratio) the amount of a solution containing auraptene adsorbed on an adsorbent (for instance, pericarp oil).

Thereafter, the auraptene is eluted from the adsorbent using an aqueous alcohol solution. Since this eluate contains auraptene, it may be used directly or may be used after being concentrated or dried. The auraptene thus obtained is substantially white, tasteless, and odorless. The alcohol concentration of the aqueous alcohol solution used for elution is, for example, 50% to 95%, preferably 60% to 90%, and more preferably 75% to 85%. The aqueous alcohol solution and the amount thereof to be added are, for example, the same as described above.

Next, an example of the present invention is described, but the present invention is not limited to the following example.

### [Example 1]

In this example, the suppression of the expression of MCP-1 at a genetic level and a protein level by auraptene was confirmed.

### <Preadipocyte differentiation induction>

First, two types of culture media indicated below, i.e. a differentiation induction culture medium and a differentiation enhancing culture medium, were prepared.

### Differentiation induction culture medium

### (Composition: 0.25 µM DEX, 0.5 mM MIX, 10 µg/mL insulin /10% FBS-containing DMEM)

First, 55 mL of FBS (fetal bovine serum (manufactured by GIBCO)) was added to 500 mL of DMEM (manufactured by SIGMA), and thereby 10% FBS-containing DMEM was prepared. Thereafter, 138.75 µL of 1 mM DEX (dexamethasone)-containing DMSO (manufactured by Nacalai Tesque, Inc.) and 555 µL of 10 mg/mL insulin-containing PBS (manufactured by SIGMA) were added thereto. The insulin-containing PBS was prepared as follows. That is, 1N HCl was added to PBS beforehand, so that PBS acidified to a degree that allowed insulin to dissolve therein, and thereafter insulin was dissolved therein. MIX (3-Isobutyl-1-methylxanthine) (manufactured by Nacalai Tesque, Inc.) was added to a required amount of the culture medium immediately before use so as to be 0.5 mM. Thus a differentiation induction culture medium was prepared. Since MIX was very difficult to dissolve, it first was dissolved in a small amount of 99.5% ethanol, which was then added to 10% FBS-containing DMEM. In this case, the final concentration of 99.5% ethanol in the differentiation induction culture medium was adjusted so as not to exceed 1%.

### Differentiation enhancing culture medium

### (Composition: 5 ug/mL insulin/10% FBS-containing DMEM/auraptene)

First, 277.5 µL of the aforementioned 10 mg/mL insulin-containing PBS was added to 555 mL of 10% FBS-containing DMEM. Furthermore, auraptene (AUR) with various concentrations was added to this mixture and thereby differentiation enhancing culture media were prepared. In this case, a cultured cell obtained by using auraptene-free differentiation enhancing culture medium containing DMSO added instead of auraptene was employed as a non-treated control (control).

Subsequently, frozen cultured preadipocyte 3T3-L1 was thawed and was plated on the culture medium placed in a 100-mm dish. This was cultured until 3T3-L1 reached about 80% confluent. One dish of 3T3-L1 that had reached about 80% confluent was subcultured on a 6-well plate. Subsequently, after 3T3-L1 subcultured on the 6-well plate further was cultured until it reached 100% confluent, the culture medium was replaced by a differentiation induction culture medium and thereby differentiation was induced. Subsequently, 48 hours after replacement by a differentiation induction culture medium, the culture medium was replaced by a differentiation enhancing culture medium. Thereafter, replacement of the differentiation enhancing culture medium was carried out every three days. Using Sepasol RNA I Super (trade name, manufactured by Nacalai Tesque, Inc.), mRNA was extracted from the cultured cells eight days after the start of differentiation induction. Subsequently, using a Light/Cycler (registered trademark), the levels of mRNA expression of MCP-1 and 36B4, which was an indicator of an early period of adipocyte differentiation, were measured. The measurement of the levels of mRNA expression is described below in detail.

Furthermore, the culture medium obtained eight days after the start of differentiation induction was collected in an amount of 1 mL from each well. Using an ELISA kit (manufactured by BD Bioscience, trade name: BD OptEIA Set Mouse MCP-1), the amount of MCP-1 (protein abundance) in the conditioned medium was measured according to the manual of the kit.

### <Quantitative determination of mRNA with Light Cycler (registered trademark)>

### Extraction and quantitative determination of total RNA

As described above, the culture medium was removed from each well of the 6-well plate, 1 mL each of Sepasol RNA I Super (manufactured by Nacalai Tesque, Inc.) was added into each well, and pipetting then was repeated for several times to disperse cells. This cell dispersion was transferred into a 1.5-mL volume tube and was then allowed to stand for five minutes at room temperature. Thereafter, 200 µL of chloroform was added into the tube, which was then stirred well with a Vortex. This was allowed to stand for three minutes at room temperature. Subsequently, the tube was cooled to 4°C, and it was centrifuged at 12000×g for 15 minutes. Thus, the content in the tube was separated into a phenol layer (lower layer; yellow) and an aqueous layer (upper layer; colorless). With care taken so as not to disturb the interface between the aforementioned layers, the aqueous layer alone was transferred into another 1.5-ml volume tube. In this case, care was taken so that the protein floating between the layers was not contained therein. Thereafter, 500 µL of isopropanol was added to the fractionated aqueous layer in the tube, which was then mixed. This was allowed to stand for ten minutes at room temperature. The tube was cooled to 4°C, and it was centrifuged at 12000×g for ten minutes. About 1 mL of supernatant was then removed. Thereafter, 1 mL of 75% ethanol was added to the recovered precipitation. This was stirred and thereby the precipitation was suspended well. Subsequently, the tube was cooled to 4°C, and it was centrifuged at 12000×g for ten minutes. The supernatant was then removed. After the precipitation (total RNA) thus obtained was dried, it was dissolved in 20 µL of water (nuclease-free water) and the concentration of mRNA then was measured with a NanoDrop (manufactured by Scrum Inc.).

### Reverse transcription

As described above, the mRNA solution whose concentration had been measured was adjusted so as to have an mRNA concentration of 1 µg/µL. Subsequently, 1 µL of oligo dT primer and 10 µL of the aforementioned RNA solution were added into 8-tube strips (with a volume of 0.2 mL). This RNA sample-primer mixture was incubated in a Thermal Cycler at 70°C for ten minutes, and thereby the conformation of RNA was destroyed. This was transferred onto ice and was allowed to stand for at least one minute. Subsequently, 11 µL of the aforementioned RNA sample-primer mixture, 5 µL of 5× reverse transcription buffer, 1 µL of RNase inhibitor, 5 µL of 2.5 mM dNTP Mix, and 2 µL of nuclease-free water were added sequentially (24 µL in total).

After the aforementioned mixture was preincubated at 42°C for five minutes in the Thermal Cycler (registered trademark), 1 µL of reverse transcriptase was added and the content in the tube was mixed well by pipetting. This was incubated at 42°C for 50 minutes and further at 70°C for 15 minutes in the Thermal Cycler. After incubation, the tube was cooled on ice, which then was centrifuged lightly and thereby the reaction solution inside the tube was gathered on the bottom of the tube. This was cryopreserved at -20°C. It was used after being diluted 10 times each time it was used for the measurement using the Light Cycler (registered trademark). This is referred to as a cDNA sample for measurement.

### Measurement using Light Cycler (registered trademark)

All the following operations were carried out inside a clean bench. A plasmid containing a fragment of a target gene (MCP-1 gene or 36B4 gene) whose expression level was to be measured was prepared as an external standard by a conventionally known method. Subsequently, 5 µL of this plasmid solution was placed in a 0.65-mL volume tube, and 45 µL of water provided in Light Cycler (registered trademark) DNA Master SYBR Green (trade name) further was added to dilute the aforementioned plasmid ten times. This operation was repeated and respective dilute solutions obtained by diluting the plasmid 10², 10³, 10⁴, 10⁵, 10⁶, 10⁷, and 10⁸ times were produced. A dedicated capillary was set on a Light Cycler (registered trademark) Centrifuge Adapter (trade name) using tweezers, and 18 µL of the mixture (24 µL in total) prepared in "Reverse transcription" described above then was dispensed into the capillary. Thereafter, further 2 µL of each of the seven types of plasmid dilute solutions and 2 µL of the aforementioned cDNA sample for measurement diluted ten times were added to the capillary, and it then was covered using tweezers. Subsequently, the capillary was centrifuged at 5000 rpm and 4°C for 10 seconds. Thereafter, this was loaded on a carousel and was set in a chamber. Measurement then was carried out. The same measurement was carried out using water instead of the aforementioned cDNA sample for measurement, as a negative control.

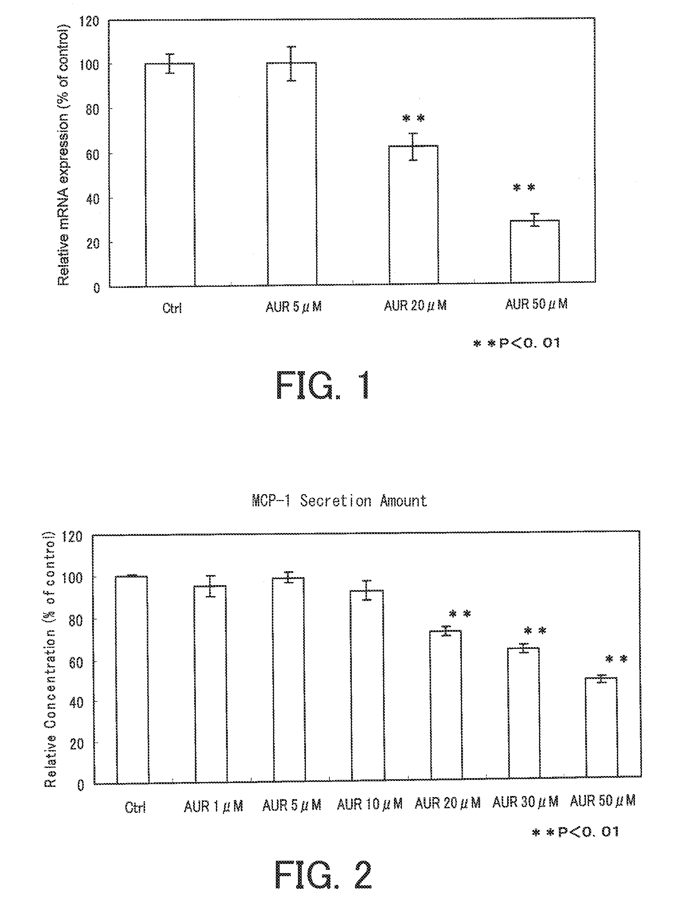

Based on the results of quantitative determination performed with the Light Cycler (registered trademark), first, the ratio (B/A) between the mRNA expression level (A) of 36B4 and the mRNA expression level (B) of MCP-1 was calculated with respect to each cDNA sample for measurement. Thereafter, with the ratio (B/A) with respect to an auraptene-free, non-treated control (control) being taken as 100%, the relative value of each sample was calculated. The results are indicated in Table 1 below and in the graph shown in FIG. 1. The auraptene concentrations indicated in Table 1 below and FIG. 1 are final concentrations in the differentiation enhancing culture media (the same applies below). In FIG. 1, AUR denotes auraptene, and Ctrl indicates the non-treated control (control). Furthermore, in FIG. 1, each amount (µM) of auraptene (AUR) added was indicated on the horizontal axis, while the relative mRNA expression level (%) to the non-treated control (control) is indicated on the vertical axis.

The relative ratios calculated, with the measured value of the non-treated control (control) with respect to the measured value of the secretion amount of MCP-1 by ELISA being taken as 100, are indicated in Table 2 below and in the graph shown in FIG. 2. The auraptene concentrations indicated in Table 2 below and FIG. 2 are final concentrations in the differentiation enhancing culture media. In FIG. 2, AUR denotes auraptene, and Ctrl indicates the non-treated control (control). Furthermore, in FIG. 2, each amount (µM) of auraptene (AUR) added was indicated on the horizontal axis, while the relative protein abundance (secretion amount) (%) of MCP-1 with respect to the non-treated control (control) is indicated on the vertical axis.

**[Table 1]**

| | Concentration of auraptene added (µM) | mRNA concentration of MCP-1 (%) |
|---|---|---|
| Non-treated control (DMSO) | 0 | 100 ± 4.3 |
| Example 1 | 5 | 99.8 ± 8.0 |
| | 20 | 62.3 ± 5.8 |
| | 50 | 28.3 ± 2.9 |
| | | (Mean value ± Standard error) |

**[Table 2]**

| | Concentration of auraptene added (µM) | Protein secretion amount of MCP-1 (%) |
|---|---|---|
| Non-treated control (DMSO) | 0 | 100 ± 0.4 |
| Example 1 | 1 | 94.6 ± 4.9 |
| | 5 | 98.6 ± 2.5 |
| | 10 | 92.1 ± 4.6 |
| | 20 | 72.3 ± 2.2 |
| | 30 | 63.9 ± 2.5 |
| | 50 | 48.5 ± 2.1 |
| | | (Mean value ± Standard error) |

As is clear from Table 1 and the graph shown in FIG. 1, it is understood that addition of auraptene made it possible to auraptene dose-dependently suppress the expression of MCP-1 at a genetic level. Furthermore, as shown in Table 2 and the graph in FIG. 2, it is understood that addition of auraptene made it possible to auraptene dose-dependently suppress the expression of MCP-1 at a protein level.

### Industrial Applicability

As described above, the suppressor of the expression of MCP-1 according to the present invention contains auraptene and thereby is excellent in safety, causes no problem even being ingested over a long period of time, and can suppress the expression of MCP-1. Furthermore, since it also has no problem in flavor, even when applied to, for example, foods, it does not impair the flavor. Accordingly, the suppressor of the expression of MCP-1 according to the present invention can be used for various products such as pharmaceuticals, supplements, foods, beverages, and food additives. The applications thereof are not limited and are wide.

## Claims

1. A suppressor of expression of MCP-1,
wherein the suppressor comprises auraptene.

2. The suppressor of expression of MCP-1 according to claim 1, wherein the suppression of expression of MCP-1 is at least one of suppression of expression at a genetic level and that at a protein level.

3. The suppressor of expression of MCP-1 according to claim 1, wherein the auraptene is auraptene derived from a citrus fruit.

4. The suppressor of expression of MCP-1 according to claim 3, wherein the citrus fruit is at least one selected from the group consisting of Amanatsu, Hassaku, Natsumikan, and grapefruit.

5. An ameliorating agent for an inflammatory disease,
wherein the ameliorating agent comprises a suppressor of expression of MCP-1 according to claim 1.

6. The ameliorating agent for an inflammatory disease according to claim 5, wherein the inflammatory disease is at least one selected from the group consisting of metabolic syndrome, allergy, renal disease, infection, autoimmune disease, rheumatoid arthritis, multiple sclerosis, organ transplant rejection, and cancer metastasis.

7. The ameliorating agent according to claim 6, wherein the metabolic syndrome is at least one selected from the group consisting of insulin resistance, hyperinsulinemia, type 2 diabetes mellitus, hyperlipidemia, arteriosclerosis, hypertension, obesity, and visceral fat accumulation.

8. A pharmaceutical for preventing or treating an inflammatory disease,
wherein the pharmaceutical comprises an ameliorating agent for an inflammatory disease according to claim 5.

9. A supplement for preventing or ameliorating an inflammatory disease,
wherein the supplement comprises an ameliorating agent for an inflammatory disease according to claim 5.

10. A food for preventing or ameliorating an inflammatory disease,
wherein the food comprises an ameliorating agent for an inflammatory disease according to claim 5.

11. A beverage for preventing or ameliorating an inflammatory disease,
wherein the beverage comprises an ameliorating agent for an inflammatory disease according to claim 5.

12. A food additive for preventing or ameliorating an inflammatory disease,
wherein the food additive comprises an ameliorating agent for an inflammatory disease according to claim 5.

13. A method of suppressing expression of MCP-1,
wherein the method comprises administering auraptene.

14. The method of suppressing expression of MCP-1 according to claim 13,
wherein the auraptene is administered by at least one method selected from the group consisting of oral administration, intraoral administration, tracheobronchial administration, rectal administration, subcutaneous administration, intramuscular administration, and intravenous administration.

15. A method of preventing or treating an inflammatory disease,
wherein the method comprises administering auraptene.

16. The method of preventing or treating an inflammatory disease according to claim 15,
wherein the auraptene is administered by at least one method selected from the group consisting of oral administration, intraoral administration, tracheobronchial administration, rectal administration, subcutaneous administration, intramuscular administration, and intravenous administration.

17. Use of auraptene for suppressing expression of MCP-1.

18. Use of auraptene for preventing or treating an inflammatory disease.
